# EUROPEAN PATENT SPECIFICATION

(11) **EP 0 978 570 B1**
(45) Date of publication and mention of the grant of the patent: **29.11.2006**
(21) Application number: 99121906.4
(22) Date of filing: 04.08.1993
(51) Int. Cl.: C12Q 1/68, C12Q 1/70, C12N 15/10, C12R 1/32, C12R 1/36

(54) **Detecting mycobacterium tuberculosis by nucleic acid sequence amplification**
Nachweis von Mycobacterium tuberculosis durch Vervielfältigung von Nukleinsäuresequenzen
Détection de mycobacterium tuberculosis par amplification de séquences d'acide nucléique

(30) Priority: 04.08.1992 US 925405
(43) Date of publication of application: 09.02.2000
(62) Divisional of application: 93306169.9
(73) Proprietor: GEN-PROBE INCORPORATED, San Diego, CA 92121 (US)
(72) Inventor: MCALLISTER, Diane L., San Diego, CA 92123 (US); HAMMOND, Philip W., Monrovia, CA 91016 (US); YEASING, Y. Yang, San Diego, CA 92130 (US); MCDONOUGH, Sherrol, San Diego, CA 92122 (US); KACIAN, Daniel, San Diego, CA 92124 (US); DATTAGUPTA, Nanibhushan, San Diego, CA 92130 (US); RYDER, Thomas B., CA 95030 (US)
(74) Representative: Irvine, Jonquil Claire

(56) References cited:
- EP-A- 0 300 796
- EP-A- 0 408 295
- EP-A- 0 587 266
- WO-A-88/03957
- WO-A-88/10315
- WO-A-93/04201

## Description

### Field of the Invention

This invention relates to methods for increasing the number of copies of a specific nucleic acid sequence or "target sequence" of M. tuberculosis rRNA which may be present either alone or as a component, large or small, of a homogeneous or hetero-geneous mixture of nucleic acids. The mixture of nucleic acids may be that found in a sample taken for diagnostic testing, environmental testing, for research studies, for the preparation of reagents or materials, for other processes such as cloning, or for other purposes.

The selective amplification of specific nucleic acid sequences is of value in increasing the sensitivity of diagnostic and environmental assays while maintaining specificity; increasing the sensitivity, convenience, accuracy and reliability of a variety of research procedures; and providing ample supplies of specific oligonucleotides for various purposes.

The present invention is particularly suitable for application to diagnostic testing due to the convenience with which it may be practised.

### Background of the Invention

The detection and/or quantitation of specific nucleic acid sequences is an increasingly important technique for identifying and classifying microorganisms, diagnosing infectious diseases, detecting and characterizing genetic abnormalities, identifying genetic changes associated with cancer, studying genetic susceptibility to disease, and measuring response to various types of treatment. Such procedures have also found expanding uses in detecting and quantitating microorganisms in foodstuffs, environmental samples, seed stocks, and other types of material where the presence of specific microorganisms may need to be monitored. Other applications are found in the forensic sciences, anthropology, archaeology, and biology where measurement of the relatedness of nucleic acid sequences has been used to identify criminal suspects, resolve paternity disputes, construct genealogical and phylogenetic trees, and aid in classifying a variety of life forms.

A common method for detecting and quantitating specific nucleic acid sequences is nucleic acid hybridization. This method is based on the ability of two nucleic acid strands that contain complementary or essentially complementary sequences to specifically associate, under appropriate conditions, to form a double-stranded structure. To detect and/or quantitate a specific nucleic acid sequence (known as the "target sequence"), a labelled oligonucleotide (known as a "probe") is prepared that contains sequences complementary to those of the target sequence. The probe is mixed with a sample suspected of containing the target sequence, and conditions suitable for hybrid formation are created. The probe hybridizes to the target sequence if it is present in the sample. The probe-target hybrids are then separated from the single-stranded probe in one of a variety of ways. The amount of label associated with the hybrids is then measured as an indication of the amount of target sequence in the sample.

The sensitivity of nucleic acid hybridization assays is limited primarily by the specific activity of the probe, the rate and extent of the hybridization reaction, the performance of the method for separating hybridized and unhybridized probe, and the sensitivity with which the label can be detected. The most sensitive procedures may lack many of the features required for routine clinical and environmental testing such as speed, convenience, and economy. Furthermore, their sensitivities may not be sufficient for many desired applications.

As a result of the interactions among the various components and component steps of this type of assay, there is almost always an inverse relationship between sensitivity and specificity. Thus, steps taken to increase the sensitivity of the assay (such as increasing the specific activity of the probe) may result in a higher percentage of false positive test results. The linkage between sensitivity and specificity has been a significant barrier to improving the sensitivity of hybridization assays. One solution to this problem would be to specifically increase the amount of target sequence present using an amplification procedure. Amplification of a unique portion of the target sequence without amplification of a significant portion of the information encoded in the remaining sequences of the sample could give an increase in sensitivity while at the same time not compromising specificity.

A method for specifically amplifying nucleic acid sequences termed the "polymerase chain reaction" or "PCR" has been described by Mullis et al. (See U.S. patents 4,683,195, 4,683,202 and 4,800,159 and European patent applications 86302298.4, 86302299.2, and 87300203.4 and Methods in Enzymology, Volume 155, 1987, pp. 335-350.) The procedure uses repeated cycles of primer dependent nucleic acid synthesis occurring simultaneously using each strand of a complementary sequence as a template. The sequence that is amplified is defined by the locations of the primer molecules that initiate synthesis. The primers are complementary to the 3'-end portion of the target sequence or its complement and must complex with those sites in order for nucleic acid synthesis to begin. After extension product synthesis, the strands are separated, generally by thermal denaturation, before the next synthesis step. In the PCR procedure, copies of both strands of a complementary sequence are synthesized.

The strand separation step used in PCR to separate the newly synthesized strands at the conclusion of each cycle of the FCR reaction is often thermal denaturation. As a result, either a thermostable enzyme is required or new enzyme must be added between thermal denaturation steps and the initiation of the next cycle of DNA synthesis. The requirement of repeated cycling of reaction temperature between several different and extreme temperatures is a disadvantage of the PCR procedure. In order to make the PCR convenient, programmable thermal cycling instruments are required.

The PCR procedure has been coupled to RNA transcription by incorporating a promoter sequence into one of the primers used in the PCR reaction and then, after amplification by the PCR procedure for several cycles, using the double-stranded DNA as template for the transcription of single-stranded RNA. (See, e.g., Murakawa et al., DNA 7:287-295 (1988).)

Other methods for amplification of a specific nucleic acid sequence comprise a series of primer hybridization, extending and denaturing steps to provide an intermediate double stranded DNA molecule containing a promoter sequence through the use of a promoter sequence-containing primer. The double stranded DNA is used to produce multiple RNA copies of the target sequence. The resulting RNA copies can be used as target sequences to produce further copies, and multiple cycles can be performed. (sometimes called "transcription amplification system" or TAS; see, e.g., Burg, et al., WO 89/1050; Gingeras, et al., WO 88/10315; European Patent Application No. 89313154.0 as published under no. 0373960 (Gingeras et al.); European Patent Application No. 88113948.9 as published under no. 0329822 (Davey and Malek); Malek et al. WO91/02818 and European Patent Application no. 90307503.4 as published under no. 0408295 (Kacian and Fultz), also discussed below.)

Walker, et al., Proc. Natl. Acad. Sci. (USA) 89:392-396 (Jan 1992) describes an oligonucleotide driven amplification method for use with DNA template, using a restriction endonuclease to produce the initial target sequences and an enzyme to nick the DNA/DNA complex in order to enable an extension reaction and therefore amplification. European Patent Application No. 88306717.5 as published under no. 0300796 (Becker et al.) describes an amplification method in which a primer is hybridized to the target sequence and the resulting duplex is cleaved prior to the extension reaction and amplification; in the case where the primer extends past the region of hybridization, it requires cleavage prior to the extension and the primer must be blocked at its 3'-end to prevent any unwanted extension reactions from occurring prior to amplification. Urdea, WO 91/10746, describes a signal amplification method that incorporates a T7 promoter sequence.

Other methods of amplifying nucleic acid include the ligase chain reaction (LCR), described in European Patent Application No. 320,308, in which at least four separate oligoprobes are used; two of the oligoprobes hybridize to opposite ends of the same target strand in appropriate orientation such that the third and fourth oligoprobes may hybridize with the first and second oligoprobes to form, upon ligation, connected probes that can be denatured and detected. Another method is that described in EP-A-0 427 073 published May 15, 1991 in which a palindromic probe able to form a hairpin and having a functional promoter region in the hairpin is hybridized to a target sequence, then ligated to another oligonucleotide hybridized to the target sequence such that specific RNA transcripts may be made.

Relatively large amounts of certain RNAs may be made using a recombinant single-stranded RNA molecule having a recognition sequence for the binding of an RNA-directed polymerase, preferably Cβ replicase. (See, e.g., U.S. Patent No. 4,786,600 to Kramer, et al.) A number of steps are required to insert the specific sequence into a DNA copy of the variant molecule, clone it into an expression vector, transcribe it into RNA and then replicate it with Qβ replicase.

### Definitions

As used herein, the following terms have the following meanings unless expressly indicated to the contrary.

### A. Nucleic Acid.

"Nucleic acid" means either RNA or DNA, along with any nucleotide analogues or other molecules that may be present in the sequence and that do not prevent performance of the present invention.

### B. Template.

A "template" is a nucleic acid molecule that is able to be copied by a nucleic acid polymerase. A template may be either RNA or DNA, and may be any of single-stranded, double-stranded or partially double-stranded, depending on the polymerase. The synthesized copy is complementary to the template. In this invention, the term copies also includes nucleic acid having the equivalent RNA or DNA sequence to a template, which are commonly referred to as homologous sequences in the art.

### C. Primer.

A "primer" is an oligonucleotide that is complementary to a template that hybridizes with the template to give a primer/template complex for initiation of synthesis by a DNA polymerase, such as a reverse transcriptase, and which is extended by the addition of covalently bonded bases linked to its 3' end that are complementary to the template. The result is a primer extension product. Virtually all DNA polymerases (including reverse transcriptases) that are known require complexing of an oligonucleotide to a single-stranded template ("priming") to initiate DNA synthesis. Under appropriate circumstances, a primer may be a part of a promoter-primer. Such primers are generally between 10 and 100 bases in length, preferably between 20 and 50 bases in length.

### D. Promoter or Promoter Sequence.

A "promoter" or "promoter sequence" is a specific nucleic acid sequence that is recognized by a DNA-dependent RNA polymerase ("transcriptase") as a signal to bind to a nucleic acid molecule and begin the transcription of RNA at a specific site. For binding, such transcriptases generally require that the promoter and its complement be double-stranded; the template portion need not be double-stranded. Individual DNA-dependent RNA polymerases recognize a variety of different promoter sequences that can vary markedly in their efficiency of promoting transcription. When an RNA polymerase binds to a promoter sequence to initiate transcription, that promoter sequence is not part of the sequence transcribed. Thus, the RNA transcripts produced thereby will not include the promoter sequence.

### E. Promoter-primer.

A promoter-primer comprises a promoter and a primer. It is an oligonucleotide that is sufficiently complementary to the 3'-end of a target nucleic acid sequence to complex at or near the 3'-end of that target nucleic acid sequence, which means that the promoter-primer complexes near enough the end of the target sequence to allow amplification of enough of the target sequence that the requirements of the assay, testing, cloning or other use for the amplified nucleic acid are met. The promoter-primer is used as a template to create a complementary nucleic acid sequence extending from the 3'-end (also known as the 3' terminus) of a target nucleic acid sequence, to result in a generally double stranded promoter, subject to any denaturing or enzymatic activity that may disrupt the double strand. Such promoter-primers are generally between 40 and 100 bases in length, preferably between 40 and 60 bases.

A DNA- or RNA-dependent DNA polymerase also creates a complementary strand to the target nucleic acid molecule, using the target sequence as a template.

### F. Modified Primer or Promoter -primer.

The 3'-end of the primer or promoter-primer may be modified, or blocked, so as to prevent or reduce the rate and/or extent of an extension reaction from proceeding therefrom. A primer or promoter-primer having both modified and unmodified members consists of essentially the same nucleic acid sequence for the purposes of the present invention. In other words, the modified primer or promoter-primer does not contain a different complexing sequence (primer) in that both the modified and unmodified oligonucleotide hybridize in effectively the same position (plus or minus about ten bases) on the target nucleic acid sequence. Also, the modified promoter-primer does not contain a different recognition sequence (promoter) from the unmodified promoter-primer. This means that, within about 10 bases, the modified and unmodified primers or promoter-primers are the same, are recognized by the same RNA polymerase, and hybridize to more or less the same target sequence (although not necessarily at precisely the same position). In a preferred embodiment, the modified and unmodified primers or promoter-primers are identical except for the modification.

The 3'-end of the target complementary portion of a primer or promoter-primer can be modified in a variety of ways well known to those skilled in the art. Appropriate modifications to a promoter-primer can include addition of ribonucleotides, 3' deoxynucleotide residues, (e.g., cordycepin (CO, Glen Research)), 3',2'-dideoxy nucleotide residues, modified nucleotides with nonphosphodiester backbone linkages (such as phosphorothioates), and non-nucleotide linkages such as described in Arnold, et al., WO 89/02439 (RS) or alkane-diol modifications (Wilk et al. Nuc. Acids Res. 18:2065, 1990) (RP), or the modification may simply consist of one or more nucleotide residues 3' to the hybridizing sequence that are uncomple-mentary to the target nucleic acid. Of course, other effective modifications are possible as well.

A mixture of modified and unmodified oligonucleotides may be used in an amplification reaction, and a broad range of ratios of modified to unmodified oligonucleotide (e.g., from 1:1 to 1,000:1) can be used. A mixture of oligonucleotides with different 3' modifications may also be used.

### G. Plus (+) and Minus (-) Strand(s).

Discussions of nucleic acid synthesis are greatly simplified and clarified by adopting terms to name the two complementary strands of a nucleic acid duplex. Traditionally, the strand encoding the sequences used to produce proteins or structural RNAs was designated as the "plus" strand and its complement the "minus" strand. It is now known that in many cases, both strands are functional, and the assignment of the designation "plus" to one and "minus"to the other must then be arbitrary. Nevertheless, the terms are very useful for designating the sequence orientation of nucleic acids and will be employed herein for that purpose, with the "plus" strand denominating the original target sequence strand that is complexed with the first primer or promoter-primer.

### H. Target Nucleic Acid Sequence, Target Sequence.

A "target nucleic acid sequence," or "target sequence," has a desired nucleic acid sequence to be amplified, and may be either single-stranded or double-stranded and may include other sequences 5' or 3' of the sequences to be amplified which may or may not be amplified.

The target nucleic acid sequence includes the complexing sequences to which the promoter-primer hybridizes during performance of the present invention. Where the target nucleic acid sequence is originally single-stranded, the term refers to either the (+) or (-) strand, and will also refer to the sequence complementary to the target sequence . Where the target nucleic acid sequence is originally double-stranded, the term refers to both the (+) and (-) strands.

### I. DNA-Dependent DNA Polymerase.

A "DNA-dependent DNA polymerase" is an enzyme that synthesizes a complementary DNA copy from a DNA template. An example is bacteriophage T7 DNA polymerase. All known DNA-dependent DNA polymerases require a complementary primer, which can be RNA or DNA, or a copolymer, to initiate synthesis. It is known that under suitable conditions certain DNA-dependent DNA polymerases may synthesize a complementary DNA copy from an RNA template.

### J. DNA-Dependent RNA Polymerase (Transcriptase).

A "DNA-dependent RNA polymerase" or "transcriptase" is an enzyme that synthesizes multiple RNA copies from a double-stranded or partially-double stranded DNA molecule having a (usually double-stranded) promoter sequence. It should be noted that the present invention includes single stranded promoter sequences in the promoter-primer, along with the RNA polymerases that recognize them. The RNA molecules ("transcripts") are synthesized in the 5' → 3' direction of the RNA molecule, beginning at a specific position just downstream of the promoter. Examples of transcriptases are the DNA-dependent RNA polymerases from bacteriophages T7, T3, and SP6.

### K. RNA-Dependent DNA Polymerase (Reverse Transcriptase).

An "RNA-dependent DNA polymerase" or "reverse transcriptase" is an enzyme that synthesizes a complementary DNA copy from an RNA template. All known reverse transcriptases also have the ability to make a complementary DNA copy from a DNA template; thus, they are both RNA- and DNA-dependent DNA polymerases. A primer is required to initiate synthesis with either the RNA or DNA templates.

### L. RNAse H.

An "RNAse H" is an enzyme that degrades the RNA portion of an RNA:DNA duplex. RNAse H's may be endo-nucleases or exonucleases. Avian myeloblastosis virus and Moloney murine leukemia virus reverse transcriptases contain an RNAse H activity in addition to their polymerase activity. Some cloned reverse transcriptases lack RNAse H activity. There are also sources of RNAse H available without an associated polymerase activity. The degradation may result in separation of RNA from an RNA:DNA complex. Alternatively, the RNAse H may simply cut the RNA at various locations such that portions of the RNA melt off or permit enzymes to unwind portions of the RNA, or the RNA fragments generated may serve as primers for extension by a polymerase.

### M. Hybridize, Complex.

The terms "hybridize" and "complex" refer to the formation of duplexes between nucleotide sequences that are sufficiently complementary to form duplexes (or "complexes") via Watson-Crick base pairing. Where a promoter-primer or primer "hybridizes" with target (template), such complexes (or hybrids) are sufficiently stable to serve the priming function required by a DNA polymerase to initiate DNA synthesis.

### N. Specificity

Specificity is a characteristic of a nucleic acid sequence that describes its ability to distinguish between target and non- target sequences, dependent on sequence and assay conditions.

### Summary of the Invention

The present invention provides a kit containing a first oligonucleotide consisting of the sequence xGCCGTCACCCCACCAACAAGCT (SEQ ID No. 22), and a second oligonucleotide consisting of the sequence

xGGGATAAGCCTGGGAAACTGGGTCTAATACC (SEQ ID No. 2), wherein x is nothing or is a sequence recognized by an RNA polymerase. Such a kit may be employed to carry out an auto-catalytic method of synthesizing multiple copies of the target M. tuberculosis rRNA nucleic acid sequence (i.e., the method cycles automatically without the need to modify reaction condi-tions such as temperature, pH, or ionic strength).

Such a method features treating a target sequence with a first oligonucleotide (that has a complex-ing sequence sufficiently complementary to a 3'-end portion of the target sequence to hybridize therewith (this alone is termed a primer), and that has a sequence 5' to the complexing sequence that includes a sequence which, in double-stranded form, acts as a promoter for an RNA polymerase (this arrangement is termed a promoter-primer)), and a second oligonucleotide (which is a primer or promoter-primer that has a complexing sequence sufficiently complementary to the complement of the target sequence to hybridize therewith), under conditions in which an oligonucleotide/target sequence complex may be formed and DNA and RNA synthesis may occur. In an embodiment, one or both of the first and second oligonucleotides is a mixture of a blocked and an unblocked oligonucleotide sequence (blocked oligonucleotides have a modified 3' end to prevent or reduce the rate and/or extent of primer extension by a DNA polymerase), or a mixture of oligonucleotides with different 3' modifications. Such a mixture significantly enhances the efficiency of the specific amplification reaction compared to use of only blocked or only unblocked oligonucleotides. The ratio of such oligonucleotides can be varied dependent upon the specific template sequence to be amplified, but generally is between 1:1 and 1000:1 blocked to unblocked. There is no requirement that the target sequence have defined 3'- or 5'- ends.

More specifically, an amplification method as described above may include (a) treating a target sequence with a first promoter-primer oligonucleo-tide that has a complexing sequence sufficiently complementary to a 3'-end portion of the target sequence to hybridize therewith, and that has a sequence 5' to the complexing sequence that includes a sequence which, in double- stranded form, acts as a promoter for an RNA poly-merase, under conditions in which an oligonucleotide/ target sequence complex may be formed and DNA synthesis may be initiated by an appropriate polymerase (e.g., a DNA polymerase), (b) incubating the first oligonucleotide/ target complex under extension reaction conditions so that the 3'-end of the target may be extended to produce a hybrid template for an RNA polymerase; and (c) incubating the hybrid template under conditions in which multiple RNA copies of the target sequence may be produced using an RNA polymerase that recognizes the promoter sequence. The invention also includes generation of a 3'-end of an RNA target sequence in step (b) by the action of an enzyme that selectively degrades the RNA portion of an RNA:DNA hybrid (e.g., RNase H). The RNA so produced may autocata-lytically cycle to produce more product.

In other methods, the following steps occur: (a) contacting a nucleic acid (e.g., RNA or DNA) target sequence with a first oligonucleotide primer or promoter-primer under conditions in which a first oligonucleotide/target sequence complex is formed such that DNA synthesis may be initiated by an appropriate polymerase (e.g., a DNA poly-merase), (b) incubating the first oligonucleotide under extension reaction conditions so that the target may be used by the polymerase as a template to give a first DNA extension product complementary to the target (if the first primer is not blocked); (c) if the target is an RNA molecule, separating the DNA extension product from the RNA target using an enzyme that selectively degrades the RNA target, or if the target is a DNA molecule, separating the two DNA strands (e.g., by heating at 90-100°C, or by other means); (d) contacting the DNA extension product with a second oligonucleotide that includes a primer or a promoter-primer, and that has a complexing sequence suffi-ciently complementary to the 3'-end portion of the DNA extension product to hybridize therewith under conditions in which a second oligonucleotide/extension product com-plex is formed and DNA synthesis may be initiated as above, depending on any blocking molecules on this primer. In this invention, if the first oligonucleotide is not a promoter-primer, then the second oligonucleotide is a promoter-primer, which means the second oligonucleotide has a sequence 5' to the complexing sequence that includes a promoter sequence for an RNA polymerase. In addition, the first and/or second oligonucleotides consist of either a mixture of a blocked and an unblocked oligonucleotide, or a mixture of oligonucleotides with different 3' modifications.

The amplification reaction is performed in a mixture consisting essentially of the necessary reactants and reagents. However, such a mixture may also contain enzymes or other substituents that do not qualitatively affect the amplification of the invention (e.g., the mechanism of the reaction). Such substituents may affect the amount of amplification observed. For example, the mixture may contain other promoter-primers for the same target sequence, or may contain "helper" oligonucleotides. Such helper oligonucleotides are used in a manner similar to the hybridization helper probes described by Hogan et al., U.S. Patent 5,030,557

namely by aiding binding of the promoter-primer to its target nucleic acid, even if that target nucleic acid has significant secondary structure. Despite the similarity in use of such helper oligonucleotides, it was surprising that such helper oligonucleotides could be used in an amplification protocol without adverse effect on the efficiency of the procedure.

The first oligonucleotide may be a promoter-primer and the second oligonucleotide may be a primer, or vice versa, or both the first and second oligonucleotides may be promoter-primers, with either identical promoters (in the sense that the promoters are recognized by the same RNA polymerase) or different promoters. Use of different promoters is particularly useful when the amplified nucleic acid will be used for cloning. The first and second oligonucleotides and the RNA produced from the target sequence may then be used to autocatalytically synthesize multiple copies (by which is meant both complementary and homologous nucleic acid sequences) of the target sequence.

The modified primer or promoter-primer of a kit of the invention consists essentially of a single nucleic acid sequence that has a modification at or near (within 3 bases) the 3'-end of the given primer or promoter- primer that alters (decreases or blocks) extension of the primer on a template by a DNA polymerase. Preferably this modified primer or promoter-primer is mixed with an unmodified primer or promoter-primer consisting essentially of the same nucleic acid sequence, along with one or more other primers or promoter-primers of a different nucleic acid sequence (that may also be a mixture of blocked and unblocked oligonucleotides). Kits of the invention may also include mixtures of primers and promoter-primers with more than one modification at or near their 3'-ends.

A kit of the invention may be employed after generation of a defined 5' end (i.e., one of known sequence) in an RNA target sequence by treating the RNA with a DNA oligonucleotide which hybridizes near the second primer binding site and thereby forms a substrate for RNAse H. This substrate is then cleaved by RNAse H to define the 5' end of the RNA target, which can be amplified as discussed above.

Amplification employing a kit of the invention may involve cooperative action of a DNA polymerase (such as a reverse transcriptase) and a DNA-dependent RNA polymerase (tran-scriptase) with an enzymatic hybrid-separation step to produce products that may themselves be used to produce additional product, thus resulting in an autocatalytic reaction without requiring manipulation of reaction conditions, such as in thermal cycling. Further, in some embodiments of the present invention that include a preliminary procedure, all but the initial step(s) of the preliminary procedure are carried out at one temperature.

In one example of a typical assay, a sample including rRNA target to be amplified is mixed with a buffer concentrate containing the buffer, salts (e.g., divalent cations such as magnesium), nucleotide triphosphates, primers and/or promoter-primers (blocked and/or unblocked), a thiol reducing agent such as dithiothreitol, and a polycation such as spermidine. The reaction is then optionally incubated near 100°C to denature any secondary structure. After cooling to room temperature (about 20°C), enzymes containing DNA and RNA dependent DNA polymerase activity, RNAse H activity and DNA dependent RNA polymerase activity are added and the mixture is incubated for about 10 minutes to four hours at 37°C to 42°C. The reaction can then be assayed by adding a luminescently-labelled probe, incubating 10 to 30 minutes at 60°C, adding a solution to selectively hydrolyze the label on unhybridized probe, incubating the reaction for 5 to 10 minutes at 60°C, and measuring the remaining chemiluminescence in a luminometer. (See, e.g., the hybridization protection assay of Arnold, *et al.* as disclosed in WO89/02476 published March 29 1989 and corresponding published European Application no. 0309230).

The kits of the invention may be used in many other assay systems known to those skilled in the art.

Yet another aspect of the invention features a mixture of modified and unmodified oligonucleotides or a mixture of differently modified oligonucleotides, said mixture being a mixture of primers or a mixture of promoter-primers having the same promoter sequence and wherein each modified or unmodified oligonucleotide hybridizes to essentially the same target sequence and wherein said oligonucleotides are selected from oligonuleotides according to any one of (a) to (c) below:
(a) an oligonucleotide consisting of a nucleic acid sequence selected from GGGATAAGCCTGGGAAACTGGGTCTAATACC (SEQ ID No. 2), and the oligonucleotide complementary to said nucleic acid sequence;
(b) an oligonucleotide as noted in (a) above joined at the 5' -end to a sequence recognized by an RNA polymerase; and
(c) an oligonucleotide as noted in (a) or (b) above which has a modification at or near its 3' -end which reduces or blocks extension by a polymerase.

It will be appreciated that such a mixture may form a component of a kit of the invention.

Kits of the invention may be diagnostic kits or other kits for use in diagnostic procedures, or other procedures, and the invention is adaptable to multi-well technology which may be provided in kit format.

### Brief Description of the Drawings

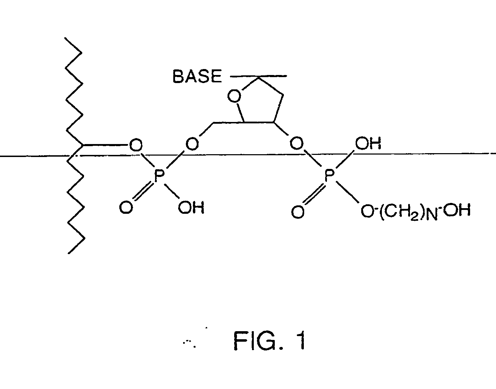

Figure 1 shows the structure of the alkane-diol modification referred to as RP.

### Detailed Description of the Invention

A kit of the invention may advantageously provide oligonucleotides for an amplification method that synthesizes RNA copies of an M. tuberculosis rRNA target sequence by use of a mixture of blocked and unblocked promoter-primers, or promoter- primers with different 3' modifications, consisting essentially of the same nucleic acid sequence in a ratio that provides for lessened non-specific by-products. The amplification process occurs spontaneously and isothermally under a broad range of conditions. The amplification reactions described below are a series of logical steps. The relative rate of each step will determine the effective yield of amplification product. Use of a mixture of blocked and unblocked primers reduces the side reactions, and hence improves amplification. Side products, such as "primer-dimers" have been described, and are well known in the art to affect the efficiency of amplification reactions. An amplification process as presented herein reduces the efficiency of formation of such byproducts, therefore enhancing amplification efficiency.

Suitable DNA polymerases include reverse transcriptases such as avian myeloblas-tosis virus (AMV) reverse transcriptase and Moloney murine leukemia virus (MMLV) reverse transcriptase. Promoters or promoter sequences suitable for incorporation in promoter-primers used in the present invention are nucleic acid sequences (either naturally occurring, produced synthetically or by a restriction endonuclease digest) that are specifically recognized by an RNA polymerase that recog-nizes and binds to that sequence and initiates the process of transcription whereby RNA transcripts are produced. Promoter sequences for which there is a known and available polymerase that is capable of recognizing the initiation sequence are particularly suitable to be employed. Such promoters include those that are recognized by certain bacteriophage polymerases such as those from bacteriophage T3, T7 or SP6. The sequence may optionally include nucleotide bases extending beyond the actual recognition site for the RNA polymerase that may impart added stability or susceptibility to degradation processes or increased transcription efficiency.

Although some of the reverse transcriptases suitable for use with oligonucleotide kits of the invention have an RNAse H activity, such as AMV or MMLV reverse transcriptase, it may be preferred to add exogenous RNAse H, such as E. coli RNAse H. For example, although the Examples (see below) show that the addition of exogenous RNAse H is not required, the RNAse H activity present in AMV reverse transcriptase may be inhibited by relatively large amounts of heterologous DNA present in the reaction mixture; one solution to the problem is to add exogenous RNAse H. Another instance when added RNAse H may be required is when an oligonucleo-tide hybridizes internally (i.e., the oligonucleotide hybridizes such that target sequence nucleotides extend past both the 3' and 5' ends of the oligonucleotide) on the target RNA.

The second oligonucleotide providing a primer sequence may be blocked or modified similarly to the first oligonucleotide. In one aspect of the present invention, if the first oligonucleotide is unmodified, then the second oligonucleotide is modified. Also, if the first oligonucleotide is not a promoter-primer, then the second oligonucleotide is a promoter-primer. Further, if the first oligonucleotide is only a primer, then it may be unblocked, and the second oligonucleotide is then a promoter-primer including both blocked and unblocked constituents consisting essentially of a single nucleic acid sequence.

Surprisingly, such a mixture of blocked and unblocked oligonucleotides consisting essentially of the same nucleic acid sequence reduces the amount of non-specific product formation, and thereby increases the effectiveness of the amplification.

The RNA copies or transcripts produced may autocatalytically multiply without further manipulation.

In another aspect of the present invention, the first and second oligonucleotides are both promoter-primers, and either or both may each consist of both modified and unmodified promoter-primers. In such a case, it is preferred that both promoters are recognized by the same RNA polymerase unless it is intended to introduce the second promoter for purposes other than amplification, such as cloning. Where both oligonucleotides are promoter-primers, then transcripts complementary to both strands of the double-stranded template will be produced during the autocatalytic reaction and the number of copies of the target sequence synthesized may be enhanced.

Note that, as the first oligonucleotide (primer or promoter-primer) defines one end of the target sequence, the second oligonucleotide (primer or promoter-primer) now defines the other end; the termini may also be defined by a specific restriction endonuclease, or by other suitable means (which may include a natural 3'-end). The RNA transcripts may have different termini from the original target nucleic acid, but the sequence between the first oligonucleotide and the second oligonucleotide remains intact. The RNA transcripts so produced may automatically recycle in the above system without further manipulation. Thus, this reaction is autocatalytic.

Also note that either oligonucleotide may have nucleotide sequences 5' to its priming sequence that can result in the addition of extra nucleotide sequence to the eventually resulting double stranded DNA; the extra nucleotide sequence is not limited to a promoter sequence.

In another embodiment, the present invention may consist of a first and second oligonucleotide in which a promoter-primer is provided which consists only of a blocked oligonucleotide, or only of an unblocked oligonucleotide, or an oligonucleotide with a mixture of different modifications at or near the 3'-end.

Amplification may be performed in the presence of additives to enhance amplification. Examples such as dimethyl sulfoxide, dimethyl formamide, ethylene glycol, glycerol or zinc have been used.

The components of the reaction mixture may be added stepwise or at once. The reaction advantageously takes place under conditions suitable for maintaining the stability of reaction components, such as the component enzymes, and without requiring modification or manipulation of reaction conditions during the course of the amplification reaction.

### Examples

### Preface

The following examples demonstrate the utility of kits of the present invention. They are not limiting and should not be considered as such.

Unless otherwise specified the reaction conditions for amplification used in the following examples were 50 mM Tris-HCl, 35 mM KCl, 20 mM MgCl₂, 15 mM N-acetylcysteine, 4 mM rATP, 4 mM rCTP,4 mM rGTP, 4 mM rUTP, 1 mM dATP, 1 mM dCTP, 1 mM dGTP, 1 mM dTTP, 10% glycerol, 10% dimethyl sulfoxide, 300-600 units MMLV reverse transcriptase, 200-400 units T7 RNA polymerase, 0.15 µM each primer or promoter-primer, and specified amounts of template and enzymes in 100 µl volumes at 42°C for one hour. Dithiothreitol, spermidine and/or polyethyleneimine (PEI) may also advantageously be added to the reaction mixture.

The enzymes used in the following examples are T7 or T3 RNA polymerase and Moloney murine leukemia virus (MMLV) reverse transcriptase. Other RNA polymerases with different promoter specificities are also suitable.

The relative amplification was measured as follows. A sample of the amplification reaction mixture (usually 10 µl) was added to 100 µl of a luminescently labelled probe (for example, labelled with an acridinium ester - see HPA reference above) solution containing approximately 75 fmol probe, 0.1 M lithium succinate, pH 4.7, 2% (w/v) lithium lauryl sulfate, 15 mM aldrithiol, 20 mM EDTA, and 20 mM EGTA, and mixed. The reactions were then incubated 20 minutes at 60°C and cooled. To each hybridization reaction was added 300 µl of 0.6 M sodium borate pH 8.5, 1% TritonJx-100. The reactions were then mixed and incubated six minutes at 60°C to destroy the chemiluminescent label of the unhybridized probe. This method of destruction of the chemiluminescent label of unhybridized probe is quite specific; only a very small fraction of the unhybridized probe remains chemiluminescent. The reactions were cooled and the remaining chemiluminescence was quantified in a luminometer upon the addition of 200 µl 0.1% hydrogen peroxide, 1 mM nitric acid, and surfactant, and 200 µl 1.0 N sodium hydroxide. In the assay, hybridized probe emits light. The quantity of photons emitted are measured in a luminometer and the results are reported as Relative Light Units or RLU. Since the reaction that destroys the chemiluminescent label of unhybridized probe is not 100% effective, there is generally a background level of signal present in the range of about 1000 to 2000 RLU.

Many other assay methods are also applicable, including assays employing hybridization to isotopically labeled probes, blotting techniques and electrophoresis. These reaction conditions are not necessarily optimized, and have been changed as noted for some systems. The oligonucleotide sequences used are exemplary and are not meant to be limiting as other sequences have been employed for these and other target sequences.

### Example 1

To show that amplification occurred with a modified promoter-primer complementary to a sequence within an RNA target, a promoter-primer complementary to a sequence within M. tuberculosis rRNA (Seq ID No. 1; primer portion is SEQ ID No. 22) was synthesized either unmodified or with a 3' alkane diol (RP) or 3' cordycepin (CO) and incubated with a primer of the same sense as the target RNA (Seq ID No. 2) and 3 zmol of target under the conditions described above. The reac-tions were analyzed with a probe of the same sense as the target RNA (Seq ID No. 3) with helper oligonucleotides as described in Hogan (U.S. Patent 5,030,557, Means for Enhancing Nucleic Acid Hybridization, Seq ID Nos. 4 and 5). The results show that significant amplification does occur with a promoter-primer containing a 3' modification.

| Promoter-primer modification | RLU |
|---|---|
| Unmodified | 314,445 |
| 3'cordycepin | 71,382 |
| Unmodified | 683,737 |
| 3'-RP | 70,014 |

### Example 2.

To show that mixtures of modified and unmodified promoter-primers function in an amplification assay, reactions were performed with 15 pmol of the primer and a promoter-primer (see below) and assayed as described in Example 1. Three zmol of target RNA was used.

| | | Pmol | Promoter-primer | | |
|---|---|---|---|---|---|
| | | Unmodified | | CO-modified | RLU |
| Experiment 1 | +Target | 15 | | 0 | 834,902 |
| | +Target | 3 | | 12 | 971,938 |
| | -Target | 3 | | 12 | 1,456 |
| Experiment 2 | +Target | 3 | | 12 | 1,015,199 |
| | +Target | 0.1 | | 15 | 961,041 |

The results show that a mixture of blocked and unblocked oligonucleotides worked as well or better than all unblocked even at a ratio of 1:150 unblocked to blocked.

### Example 3.

In this experiment 3 zmol of target RNA were incubated with different concentrations of CO blocked and unblocked primer and a mixture of 15 pmol CO blocked promoter-primer and 0.1 pmol unblocked promoter-primer as in Example 1. Products were detected by hybridization assay.

| Pmol | Primer | RLU |
|---|---|---|
| Blocked | Unblocked | |
| 0 | 15 | 969,522 |
| 10 | 5 | 802,840 |
| 13 | 2 | 648,271 |

In addition to the satisfactory amplification observed, it was surprisingly found that the amount of non-template directed product was significantly less in the reactions performed with blocked oligonucleotides compared to reactions performed with unblocked oligonucleotides.

### Example 4.

In this experiment, the effect of mixing a single oligonucleotide sequence with two different 3' modifications was demonstrated. Three zmol of target RNA was amplified as in Example 1. The promoter-primer was synthesized with an unblocked 3'-end, blocked with RP, or CO blocked. Two pmol of primer were used.

| Pmol | Promoter-primer | | | RLU |
|---|---|---|---|---|
| | RP modified | CO modified | Unmodified | |
| | 0 | 15 | 0.1 | 450,157 |
| | 2 | 13 | 0.01 | 681,647 |
| | 2 | 13 | 0 | 678,871 |
| | 5 | 10 | 0 | 755,839 |

This example shows that a mixture of unmodified and modified or a mixture of different types of modified promoter- primers amplified well, allowing detection of 3 zmol of RNA target in one hour.

### Example 5.

In this example, a mixture of modified and unmodified primers and promoter-primers were used to amplify 3 zmol M. tuberculosis rRNA. A mixture of 2 pmol RP-modified promoter-primer and 13 pmol of CO-modified promoter-primer were incubated with unmodified primer or a mixture of unmodified primer and primer synthesized with a 3' phosphorothioate nucleotide (PS). The sequences and hybridization probes are as in Example 1.

| Primer modification | | RLU |
|---|---|---|
| Unmodified | PS modified | |
| -- | 15 pmol | 118,411 |
| 1 pmol | 14 pmol | 364,733 |
| No target | | 1,266 |

Under these conditions, the mixture of modified and unmodified primers work best.

## Claims

1. A kit containing a first oligonucleotide consisting of the sequence xGCCGTCACCCCACCAACAAGCT (SEQ ID No. 22), and a second oligonucleotide consisting of the sequence xGGGATAAGCCTGGGAAACTGGGTCTAATACC, (SEQ ID No. 2), wherein x is nothing or is a sequence recognized by an RNA polymerase.

2. A kit as claimed in claim 1 wherein one of said first and second oligonucleotides is a promoter-primer.

3. A kit as claimed in claim 2 wherein said first oligonucleotide is the oligonucleotide represented by SEQ ID NO. 1 (GAAATTAATACGACTCACTA T AGGGAGACCACAGCCGTCACCCCACCAACAAGCT) and said second oligonucleotide is the sequence represented by SEQ ID No.2 (GGGATAAGCC TGGGAAACTGGGTCTAATACC).

4. A kit as claimed in any one of claims 1 to 3 wherein one or more of said sequences has a modified 3'-end which reduces or blocks extension by a polymerase.

5. A kit as claimed in claim 2 or claim 3 wherein the promoter-primer comprises a mixture of modified and unmodified members or a mixture of differently modified members, each modified member having a modification at or near the 3'- end which reduces or blocks extension by a polymerase.

6. A kit as claimed in any one of claims 1 to 5 further comprising a hybridisation probe, said probe comprising an oligonucleotide consisting of the sequence GTCTTGTGGTGGAAAGCGTTTAG (SEQ ID No. 3).

7. A kit as claimed in any one of claims 1 to 6 further comprising helper oligonucleotides represented by SEQ No. 4 (CCGGATAGGACCACGGGATGCAT) and SEQ No.5 (CGGTGTGGGATGACCCCGCG).

8. A method for amplifying Mycobacterium nucleic acid in a sample comprising amplification of said nucleic acid with a pair of oligonucleotides selected from pairs of oligonucleotides for nucleic acid amplification as defined in any one of claims 1 to 5.

9. A method for the detection of M. tuberculosis nucleic acid comprising amplification of said nucleic acid with a pair of oligonucleotides selected from pairs of oligonucleotides for nucleic acid amplification as defined in any one of claims 1 to 5 and detection of the amplified nucleic acid with a hybridization probe as defined in claim 6.

10. Use of the set of oligonucleotides of the kit as claimed in claim 7 to detect M. tuberculosis nucleic acid by a method according to claim 9.

11. An oligonucleotide consisting of a nucleic acid sequence selected from GGGATAAGCCTGGGAAACTGGGTCTAATACC (SEQ ID No. 2), and the oligonucleotide complementary to said nucleic acid sequence.

12. An oligonucleotide as claimed in claim 11 joined at the 5' -end to a sequence recognized by an RNA polymerase.

13. An oligonucleotide as claimed in claim 11 or claim 12 which has a modification at or near its 3' -end which reduces or blocks extension by a polymerase.

14. A mixture of modified and unmodified oligonucleotides or a mixture of differently modified oligonucleotides, said mixture being a mixture of primers or a mixture of promoter-primers having the same promoter sequence and wherein each modified or unmodified oligonucleotide hybridizes to essentially the same target sequence and wherein said oligonucleotides are selected from oligonucleotides according to any one of claims 11 to 13.

## Patentansprüche

1. Kit enthaltend ein erstes Oligonucleotid bestehend aus der Sequenz xGCCGTCACCCCACCAACAAGCT (SEQ ID NO:22) und ein zweites Oligonucleotid bestehend aus der Sequenz xGGGATAAGCCTGGGAAACTGGGTCTAATACC (SEQ ID NO:2), wobei x für nichts steht oder eine durch eine RNA-Polymerase erkannte Sequenz ist.

2. Kit nach Anspruch 1, wobei eines des ersten und zweiten Oligonucleotide ein Promotorprimer ist.

3. Kit nach Anspruch 2, wobei das erste Oligonucleotid das Oligonucleotid ist, das durch SEQ ID NO:1 (GAAATTAATACGACTCACTATAGGGAGACCACAGCCGTCACCCCACCAACA AGCT) dargestellt ist, und das zweite Oligonucleotid die Sequenz ist, die durch SEQ ID NO:2 (GGGATAAGCCTGGGAAACTGGGTCTAATACC) dargestellt ist.

4. Kit nach einem der Ansprüche 1 bis 3, wobei eine oder mehrere der Sequenzen ein modifiziertes 3'-Ende aufweist bzw. aufweisen, das die Verlängerung durch eine Polymerase reduziert oder blockiert.

5. Kit nach Anspruch 2 oder Anspruch 3, wobei der Promotorprimer eine Mischung von modifizierten und unmodifizierten Gliedern oder eine Mischung von unterschiedlich modifizierten Gliedern umfasst, wobei jedes modifiziertes Glied eine Modifikation bei oder in der Nähe des 3'-Endes aufweist, das die Verlängerung durch eine Polymerase reduziert oder blockiert.

6. Kit nach einem der Ansprüche 1 bis 5, des Weiteren umfassend eine Hybridisierungssonde, wobei die Sonde ein Oligonucleotid umfasst bestehend aus der Sequenz GTCTTGTGGTGGAAAGCGTTTAG (SEQ ID NO:3).

7. Kit nach einem der Ansprüche 1 bis 6, des Weiteren umfassend Helferoligonucleotide dargestellt durch SEQ ID NO:4 (CCGGATAGGACCACGGGATGCAT) und SEQ ID NO:5 (CGGTGTGGGATGACCCCGCG).

8. Methode für das Amplifizieren der Mycobacterium-Nucleinsäure in einer Probe umfassend die Amplifikation der Nucleinsäure mit einem Paar von Oligonucleotiden ausgewählt aus Paaren von Oligonucleotiden für die Nucleinsäureamplifikation, wie in irgendeinem der Ansprüche 1 bis 5 definiert.

9. Methode zum Erfassen von M. tuberculosis-Nucleinsäure, umfassend die Amplifikation der Nucleinsäure mit einem Paar Oligonucleotiden ausgewählt aus Paaren von Oligonucleotiden für die Nucleinsäureamplifikation, wie in irgendeinem der Ansprüche 1 bis 5 definiert, und das Erfassen der amplifizierten Nucleinsäure mit einer Hybridisierungssonde, wie in Anspruch 6 definiert.

10. Verwendung eines Satzes von Oligonucleotiden des Kits nach Anspruch 7 zum Erfassen von M. tuberculosis-Nucleinsäure durch eine Methode nach Anspruch 9.

11. Oligonucleotid bestehend aus einer Nucleinsäuresequenze ausgewählt aus GGGATAAGCCTGGGAAACTGGGTCTAATACC (SEQ ID NO:2) und dem Oligonucleotid, das zu der Nucleinsäuresequenz komplementär ist.

12. Oligonucleotid nach Anspruch 11, das am 5'-Ende mit einer durch eine RNA-Polymerase erkannten Sequenz verbunden ist.

13. Oligonucleotid nach Anspruch 11 oder Anspruch 12, das eine Modifikation an oder in der Nähe seines 3'-Endes aufweist, die die Verlängerung durch eine Polymerase reduziert oder blockiert.

14. Mischung modifizierter und unmodifizierter Oligonucleotide oder Mischung unterschiedlich modifizierter Oligonucleotide, wobei die Mischung eine Mischung von Primern oder eine Mischung von Promotorprimern ist, die die gleiche Promotorsequenz aufweisen und wobei jedes modifizierte oder unmodifizierte Oligonucleotid im Wesentlichen an die gleiche Zielsequenz hybridisiert und wobei die Oligonucleotide aus den Oligonucleotiden nach einem der Ansprüche 11 bis 13 ausgewählt sind.

## Revendications

1. Kit contenant un premier oligonucléotide consistant en la séquence xGCCGTCACCCCACCAACAAGCT (SEQ ID NO:22), et un deuxième oligonucléotide consistant en la séquence xGGGATAAGCCTGGGAAACTGGGTCTAATACC (SEQ ID NO:2), où x n'est rien ou est une séquence reconnue par une ARN polymérase.

2. Kit tel que revendiqué dans la revendication 1, dans lequel l'un desdits premier et deuxième oligonucléotides est une amorce promoteur.

3. Kit tel que revendiqué dans la revendication 2, dans lequel ledit premier oligonucléotide est l'oligonucléotide représenté par la SEQ ID NO:1 (GAAATTAATACGACTCACTATAGGGAGACCACAGCCGTCACCCCACCAACA AGCT), et ledit deuxième oligonucléotide est la séquence représentée par la SEQ ID NO:2 (GGGATAAGCCTGGGAAACTGGGTCTAATACC).

4. Kit tel que revendiqué dans l'une quelconque des revendications 1 à 3, dans lequel une ou plusieurs desdites séquences ont une extrémité 3' modifiée qui réduit ou bloque l'extension par une polymérase.

5. Kit tel que revendiqué dans la revendication 2 ou la revendication 3, dans lequel l'amorce promoteur comprend un mélange de membres modifiés et non modifiés ou un mélange de membres modifiés différemment, chaque membre modifié ayant une modification à ou près de l'extrémité 3', qui réduit ou bloque l'extension par une polymérase.

6. Kit tel que revendiqué dans l'une quelconque des revendications 1 à 5, comprenant en outre une sonde d'hybridation, ladite sonde comprenant un oligonucléotide consistant en la séquence GTCTTGTGGTGGAAAGCGTTTAG (SEQ ID NO:3).

7. Kit tel que revendiqué dans l'une quelconque des revendications 1 à 6, comprenant en outre des oligonucléotides auxiliaires représentés par la SEQ ID NO:4 (CCGGATAGGACCACGGGATGCAT) et la SEQ ID NO:5 (CGGTGTGGGATGACCCCGCG).

8. Procédé d'amplification d'acide nucléique de Mycobacterium dans un échantillon, comprenant l'amplification dudit acide nucléique avec une paire d'oligonucléotides sélectionnée parmi des paires d'oligonucléotides d'amplification d'acide nucléique comme il est défini dans l'une quelconque des revendications 1 à 5.

9. Procédé de détection d'acide nucléique de M. tuberculosis, comprenant l'amplification dudit acide nucléique avec une paire d'oligonucléotides sélectionnée parmi des paires d'oligonucléotides d'amplification d'acide nucléique comme il est défini dans l'une quelconque des revendications 1 à 5, et la détection de l'acide nucléique amplifié avec une sonde d'hybridation comme il est défini dans la revendication 6.

10. Utilisation du jeu d'oligonucléotides du kit tel que revendiqué dans la revendication 7, pour détecter l'acide nucléique de M. tuberculosis par un procédé selon la revendication 9.

11. Oligonucléotide consistant en une séquence d'acide nucléique sélectionnée parmi GGGATAAGCCTGGGAAACTGGGTCTAATACC (SEQ ID NO:2) et l'oligonucléotide complémentaire à ladite séquence d'acide nucléique.

12. Oligonucléotide tel que revendiqué dans la revendication 11, raccordé à l'extrémité 5' à une séquence reconnue par une ARN polymérase.

13. Oligonucléotide tel que revendiqué dans la revendication 11 ou la revendication 12, qui a une modification à ou près de son extrémité 3', qui réduit ou bloque l'extension par une polymérase.

14. Mélange d'oligonucléotides modifiés et non modifiés ou mélange d'oligonucléotides modifiés différemment, ledit mélange étant un mélange d'amorces ou un mélange d'amorces promoteurs ayant la même séquence promoteur, et où chaque oligonucléotide modifié ou non modifié s'hybride essentiellement à la même séquence cible et où lesdits oligonucléotides sont sélectionnés parmi des oligonucléotides selon l'une quelconque des revendications 11 à 13.


### SEQUENCE LISTING

<110> Gen-Probe Incorporated
<120> DETECTING MYCOBACTERIUM TUBERCULOSIS BY NUCLEIC ACID SEQUENCE AMPLIFICATION
<130> P53256EP
<140> 99121906.4
   <141> 1993-08-04
<150> EP 93306169.9
   <151> 1993-08-04
<150> US 07/925,405
   <151> 1992-08-04
<160> 22
<170> PatentIn Ver. 2.1
<210> 1
   <211> 55
   <212> DNA
   <213> Artificial Sequence
<220>
   <223> Description of Artificial Sequence: Synthetic Oligonucleotide
<400> 1
   gaaattaata cgactcacta tagggagacc acagccgtca ccccaccaac aagct 55
<210> 2
   <211> 31
   <212> DNA
   <213> Artificial Sequence
<220>
   <223> Description of Artificial Sequence: Synthetic Oligonucleotide
<400> 2
   gggataagcc tgggaaactg ggtctaatac c 31
<210> 3
   <211> 24
   <212> DNA
   <213> Artificial Sequence
<220>
   <223> Description of Artificial Sequence: Synthetic Oligonucleotide
<400> 3
   gtcttgtggt ggaaagcgct ttag 24
<210> 4
   <211> 23
   <212> DNA
   <213> Artificial Sequence
<220>
   <223> Description of Artificial Sequence: Synthetic Oligonucleotide
<400> 4
   ccggatagga ccacgggatg cat 23
<210> 5
   <211> 20
   <212> DNA
   <213> Artificial Sequence
<220>
   <223> Description of Artificial Sequence: Synthetic Oligonucleotide
<400> 5
   cggtgtggga tgaccccgcg 20
<210> 6
   <211> 47
   <212> DNA
   <213> Artificial Sequence
<220>
   <223> Description of Artificial Sequence: Synthetic Oligonucleotide
<400> 6
   aatttaatac gactcactat agggagacca ggccacttcc gctaacc 47
<210> 7
   <211> 24
   <212 > DNA
   <213> Artificial Sequence
<220>
   <223> Description of Artificial Sequence: Synthetic Oligonucleotide
<400> 7
   cgcggaacag gctaaaccgc acgc 24
<210> 8
   <211> 23
   <212> DNA
   <213> Artificial Sequence
<220>
   <223> Description of Artificial Sequence: Synthetic Oligonucleotide
<400> 8
   ggaggatatg tctcagcgct acc 23
<210> 9
   <211> 38
   <212> DNA
   <213> Artificial Sequence
<220>
   <223> Description of Artificial Sequence: Synthetic Oligonucleotide
<400> 9
   cggctgagag gcagtacaga aagtgtcgtg gttagcgg 38
<210> 10
   <211> 36
   <212> DNA
   <213> Artificial Sequence
<220>
   <223> Description of Artificial Sequence: Synthetic Oligonucleotide
<400> 10
   gggtaaccgg gtaggggttg tgtgtgcggg gttgtg 36
<210> 11
   <211> 28
   <212> DNA
   <213> Artificial Sequence
<220>
   <223> Description of Artificial Sequence: Synthetic Oligonucleotide
<400> 11
   ataatccacc tatcccagta ggagaaat 28
<210> 12
   <211> 55
   <212> DNA
   <213> Artificial Sequence
<220>
   <223> Description of Artificial Sequence: Synthetic Oligonucleotide
<400> 12
   aatttaatac gactcactat agggagacca caccttgtct tatgtccaga atgct 55
<210> 13
   <211> 30
   <212> DNA
   <213> Artificial Sequence
<220>
   <223> Description of Artificial Sequence: Synthetic Oligonucleotide
<400> 13
   gcacgtagtt agccggtgct tattcttcag 30
<210> 14
   <211> 53
   <212> DNA
   <213> Artificial Sequence
<220>
   <223> Description of Artificial Sequence: Synthetic Oligonucleotide
<400> 14
   aatttaatac gactcactat agggagagca agcctgatcc agccatgccg cgt 53
<210> 15
   <211> 32
   <212> DNA
   <213> Artificial Sequence
<220>
   <223> Description of Artificial Sequence: Synthetic Oligonucleotide
<400> 15
   gcttgcgccc attgtccaaa atttcccact gc 32
<210> 16
   <211> 18
   <212> DNA
   <213> Artificial Sequence
<220>
   <223> Description of Artificial Sequence: Synthetic Oligonucleotide
<400> 16
   tcggccgccg atattggc 18
<210> 17
   <211> 40
   <212> DNA
   <213> Artificial Sequence
<220>
   <223> Description of Artificial Sequence: Synthetic Oligonucleotide
<400> 17
   aacggccttt tcttccctga caaaagtcct ttacaacccg 40
<210> 18
   <211> 36
   <212> DNA
   <213> Artificial Sequence
<220>
   <223> Description of Artificial Sequence: Synthetic Oligonucleotide
<400> 18
   cgtagttagc cggtgcttat tcttcaggta ccgtca 36
<210> 19
   <211> 46
   <212> DNA
   <213> Artificial Sequence
<220>
   <223> Description of Artificial Sequence: Synthetic Oligonucleotide
<400> 19
   taatattaac cctcactaaa gggagaccag gccacttccg ctaacc 46
<210> 20
   <211> 28
   <212> DNA
   <213> Artificial Sequence
<220>
   <223> Description of Artificial Sequence: Synthetic Oligonucleotide
<400> 20
   ataatccacc tatcccagta ggagaaat 28
<210> 21
   <211> 55
   <212> DNA
   <213> Artificial Sequence
<220>
   <223> Description of Artificial Sequence: Synthetic Oligonucleotide
<400> 21
   aatttaatac gactcactat agggagacca caccttgtct tatgtccaga atgct 55
<210> 22
   <211> 22
   <212> DNA
   <213> Artificial Sequence
<220>
   <223> Description of Artificial Sequence: Synthetic Oligonucleotide
<400> 22
   gccgtcaccc caccaacaag ct 22